Europäisches Patentamt
European Patent Office
Office européen des brevets

(19)

(11) Publication number: 0 193 257 A1

(12) **EUROPEAN PATENT APPLICATION**

(21) Application number: 86300284.6

(22) Date of filing: 16.01.86

(51) Int. Cl.[4]: A 01 N 33/10
A 01 N 35/04, A 61 K 7/00
A 61 K 7/06, A 61 K 7/48
A 61 K 31/11, A 61 K 31/135

(30) Priority: 23.01.85 GB 8501646

(43) Date of publication of application: 03.09.86 Bulletin 86/36

(84) Designated Contracting States: AT BE CH DE FR GB IT LI LU NL SE

(71) Applicant: NIPA LABORATORIES, LTD.
Hayes Road
Cadishead Manchester, M30 5BX(GB)

(72) Inventor: Maddox, David Norman
"Glandwr" Rudry Road
Lisvane Cardiff, CF4 5SN(GB)

(72) Inventor: Smith, Trevor Stanley
4, Melrose Houses, Toredyrhlw Merthyr Tydfil
Mid Glamorgan(GB)

(72) Inventor: Payne, Lyndon Christopher
91 Mildred Street Ty-Nant
Pontypridd Mid Glamorgan(GB)

(74) Representative: Taylor, Phillip Kenneth et al,
W.P. THOMPSON & CO. Coopers Building Church Street
Liverpool L1 3AB(GB)

(54) **Control of micro-organisms in water-containing compositions.**

(57) A method of preventing the growth of fungi and bacteria including Gram-negative bacteria in water-containing compositions characterised by the addition to the composition, as an agent for the control of Gram-negative bacteria, of one or more compounds of the general formula:

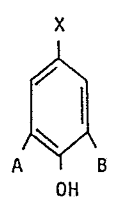

where X represents CHO or a mannic base, represented by

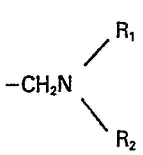

where $R_1$ and $R_2$ each represent an alkyl group (preferably containing 1 to 4 carbon atoms), or, together with an oxygen atom form the residue of an N-morpholinyl ring,

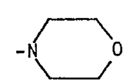

A represents fluorine, bromine, chlorine, iodine, an alkyl group containing from 1 to 4 carbon atoms, an alkoxy group containing from 1 to 4 carbon atoms, a hydroxyl group, a nitro group, a primary amino group or hydrogen;

B represents fluorine, bromine, chlorine, iodine, an alkyl group containing 1 to 4 carbon atoms, an alkoxy group containing 1 to 4 carbon atoms, a hydroxy group, a nitro group, a primary amino group or hydrogen provided that where X is a mannic base only one of A and B may represent hydrogen.

EP 0 193 257 A1

## DESCRIPTION

## CONTROL OF MICRO-ORGANISMS IN WATER-CONTAINING COMPOSITIONS.

The present invention relates to the control of micro-organisms in water-containing compositions.

Many water-containing compositions, such as detergents, solutions, emulsions, suspensions or gels, are difficult to preserve. Such compositions include liquid detergents, cutting emulsions, adhesives, paints, thickener solutions, shampoos, fabric softeners and foam baths. The water-containing compositions may contain viable bacteria, yeasts and moulds. These microbes can multiply and the colonies formed can degrade and spoil the product, such spoilage being evident as changes in odour, colour or consistency of the product. Such problems also occur in industrial cooling water systems.

Excessively high concentrations of microbes in the composition can become a health hazard. For instance, one of the most common contaminants is the highly adaptable bacterium Pseudomonas aeruginosa which is a known causative organism of eye infections, and, as such, would not be welcome in a shampoo.

Many compounds have been used as antimicrobial preservatives in toiletry, household and industrial water-containing products. Some are unsuitable for widespread use, one reason being that they are toxic hazards. The rest, such as methyl-4-hydroxybenzoate and sodium benzoate, are widely accepted as antimicrobial preservatives but, whilst they are effective against fungi and Gram positive bacteria, they are relatively inactive against Gram negative bacteria, for example Pseudmonas, Klebsiella, Citrobacter, Flavobacterium, and Serratia marcescens. The problem of microbial contamination and spoilage is

especially acute in those compositions which are not heated during production, since water-borne Gram-negative bacteria may then survive the manufacturing process, so allowing these chemically resistant bacteria to grow in the end product.

An object of the present invention is to provide a method of preserving water-containing products such as that preservative activity against Gram negative bacteria is as great as activity against other micro-organisms.

A further object is to provide a method of preserving water-containing products in which the active stabilizer is stable for long periods. A still further object is to provide such a method in which the active stabilization has low-toxicity levels.

It has now been found that a series of aromatic compounds which have properties controlling Gram-positive bacteria and fungi are at least equally as effective against Gram-negative bacteria such as those illustrated above.

According to the present invention there is provided a method of preventing the growth of fungi and bacteria including Gram-negative bacteria in water-containing compositions by the addition to the composition, as an agent for the control of Gram-negative bacteria, of one or more compounds of the general formula:

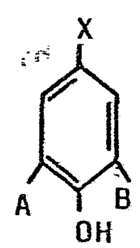

wherein X represents CHO or a mannic base, represented by:

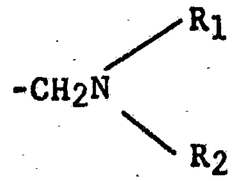

in which $R_1$ and $R_2$ each represent an alkyl group (preferably containing 1 to 4 carbon atoms), or, together with an oxygen atom form the residue of an N-morpholinyl ring,

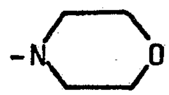

A represents fluorine, bromine, chlorine, iodine, an alkyl group containing from 1 to 4 carbon atoms, an alkoxy group containing from 1 to 4 carbon atoms, a hydroxy group, a nitro group, a primary amino group or hydrogen;

B represents fluorine, bromine, chlorine, iodine, an alkyl group containing from 1 to 4 carbon atoms, an alkoxy group containing from 1 to 4 carbon atoms, a hydroxy group, a nitro group, a primary amino group, or hydrogen;

provided that when X is a mannic base only one of A and B may be hydrogen. Preferably in all cases only one of A and B are hydrogen.

In general from about 0.01% to about 0.6% by weight of the control agent based on the total weight of the water-containing composition may be used, although larger amounts are not excluding for any specific product. Preferably from 0.02 to 0.2% by weight of the control agent based on the total weight of the water-containing composition is used.

The control agent of the present invention may be added to or incorporated in the water-containing composition as such but, especially when the control agent is normally a solid material it may be added in the form of a solution of appropriate concentration in a suitable solvent which is compatible with the water-containing composition. Thus, in the case of 3,5-dichloro-4-hydroxybenzaldehyde (DCHB) a solution of DCHB is monopropylene glycol comprising 30 parts by weight DCHB to 70 parts by weight monopropylene glycol can be used.

It has been found that compounds of the above general formula, especially DCHB have the advantage of retaining antimicrobial activity in the presence of proteins, such as serum and soluble collagen, whereas many currently available antimicrobial agents are significantly inactivated by proteins.

It is important that preservatives added to toiletry, household and pharmaceutical products have a low order of toxicity. The toxicological data which has so far been accumulated on DCHB, as an example of compounds of the above general formula, is extremely favourable as exemplified by an acute oral toxicity ($LD_{50}$) in rats of greater than 5 g/kg body weight.

It has further been found that compounds of the above general formula, especially DCHB, have excellent stability. Samples of shampoos, aqueous solutions and creams with and without addition of DCHB at 0.3% (w/w) have been stored at 25°C, in the light and in dark, for seven months and have been assessed for visual colour change and for DCHB content, by UV analysis, at regular intervals. In that time no colour changes have been noted and there has been no significant change in UV absorption readings.

The present invention also includes the use of one or more compounds of the general formula:

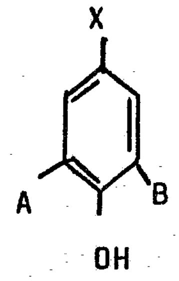

wherein X, A and B have the meanings defined hereinbefore in the preparation of a medicament for use in the control of bacteria (especially gram-negative bacteria) upon skin (especially human skin) by the application of said medicament to the skin.

Examples of such compounds which are effective in preventing the growth of Gram-negative bacteria include:

1. 3,5-Dibromo-4-hydroxybenzaldehyde

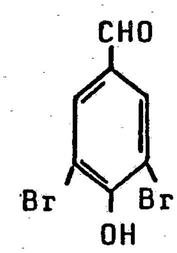

2. 3,5,-Dimethyl-4-hydroxybenzaldehyde

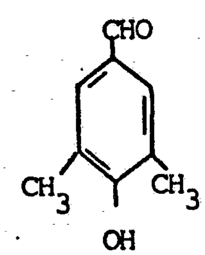

3. 3-Chloro-4-hydroxybenzaldehyde

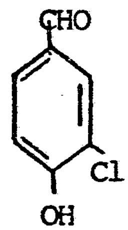

4. 3,5-Dimethoxy-4-hydroxybenzaldehyde

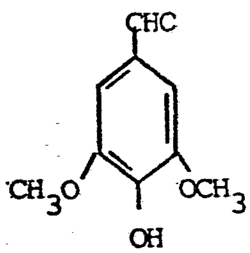

5. 2,6-Dimethoxy-4-(N-morpholinyl) methyl phenol

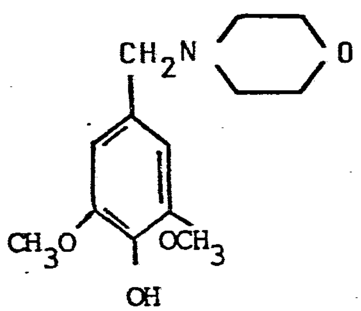

6. 3,5-Dichloro-4-hydroxybenzaldehyde

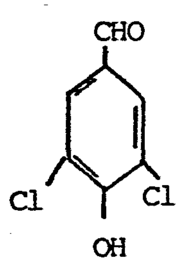

These compounds are also useful in controlling the growth of fungi and Gram-positive bacteria.

Although the compounds of the above general formula used in the method of the invention do have a controlling effect on Gram-positive bacteria and fungi, they may be used in conjunction with other anti-microbial agents effective against gram-positive bacteria and fungi, (e.g. yeasts and moulds) such as methyl-4-hydroxybenzoate. It has also been found that potentiators of antimicrobial activity such as chelating agents (e.g. citric acid or ethylene diamine tetraacetic acid) can also be effective in combination with compounds of the above general formula as used in the method of the present invention.

Specific embodiments of the invention will now be described with reference to the following Examples.

EXAMPLE 1

Sodium lauryl ether sulphate (SLES) is a commonly used anionic detergent, a major active component of shampoos, foam baths and general purpose cleaners. The bactericidal activity of some compounds covered by the general formula given above was evaluated at final levels of 0.10% (w/w) and 0.15% (w/w) in a 27% active solution of SLES (confirmed as containing no other preservatives). The activity of these compounds was evaluated in comparison with 0.30% methyl-4-hydroxybenzoate and an unpreserved control sample.

The test organisms were three species of resistant Gram negative bacteria which had previously been isolated from contaminated detergent products. 20g portions of test solutions were inoculated with 0.1 ml pure culture of the test bacteria and the time taken to achieve a total lethal effect against each species was determined by sub-culture at regular intervals into sterile dilution media (containing preservative inactivators) and then into sterile Nutrient Broth, which was incubated for 4 days at 32°C to determine the presence or absence of surviving bacteria.

| Test species | Initial inoculum level colony forming units per g |
|---|---|
| Pseudomonas aeruginosa | $8.4 \times 10^7$ |
| Serratia marcescens | $5.1 \times 10^7$ |
| Klebsiella oxytoca | $3.9 \times 10^7$ |

Results

Bactericidal effect of compounds in 27% Sodium Lauryl Ether Sulphate

| 27% SLES containing: | Time to achieve total lethal effect against: P aeruginosa | S marcescens | K oxytoca |
|---|---|---|---|
| 0.10% 3,5-Dibromo-4-hydroxybenzaldehyde | 1 hour | 3 hours | 3 hours |
| 0.15% -"- | <1 hour | <1 hour | <1 hour |
| 0.10% 3,5-Dimethyl-4-hydroxybenzaldehyde | 1 hour | 1 day | 3 hours |
| 0.15% -"- | <1 hour | 3 hours | <1 hour |
| 0.10% 3-Chloro-4-hydroxybenzaldehyde | 3 hours | 1 day | 6 hours |
| 0.15% -"- | 1 hour | 6 hours | 1 hour |
| 0.3% Methyl-4-hydroxybenzoate | 14 days | >28 days | 21 days |
| Control (no preservative) | >28 days | >28 days | >28 days |
| Colony forming units in control after 28 days (per g) | $9.3 \times 10^7$ | $2.2 \times 10^8$ | $3.9 \times 10^8$ |

EXAMPLE 2

Emulsions are susceptible to contamination and spoilage by a wide range of micro-organisms. 3.5-Dibromo-4-hydroxybenzaldehyde (DBHB), as a non-limiting illustration of the invention, was evaluated in a typical cosmetic cream for microbiocidal efficacy. The cosmetic cream had the following formulation:

| | | |
|---|---|---|
| Emulsifying wax | 16% | |
| Propylene glycol | 5% | |
| Water | 79% | PH 6-8 |

This cream was prepared by heating the oil and water phases to 70°C, mixing and stirring until cool. The DBHB was incorporated into the cream at final levels of 0.05%, 0.10% and 0.15% by dissolving into the emulsion components at the heating stage. A microbial challenge test was performed to determine the micro-biocidal activity of the test product. The following test organisms were inoculated in pure culture into portions of sterile cream samples and the time taken to achieve a total lethal effect was determined by regular sub-culture into recovery media (with preservative inactivators) which were incubated and observed for growth of survivors.

| Test species | | Initial inoculum level colony forming units per g |
|---|---|---|
| Pseudomonas aeruginosa | Gram negative | $6.4 \times 10^7$ |
| Escherichia coli | Gram negative | $9.1 \times 10^7$ |
| Serratia marcescens | Gram negative | $8.7 \times 10^7$ |
| Pseudomonas putida | Gram negative | $9.0 \times 10^6$ |
| Staphylococcus aureus | Gram positive | $6.0 \times 10^6$ |
| Candida albicans | Yeast | $5.4 \times 10^6$ |
| Pencicillium expansum | Mould | $1.0 \times 10^5$ |

Results

| Cosmetic cream containing | Time taken to achieve total lethal effect against: | | | | | | |
|---|---|---|---|---|---|---|---|
| | P aeruginosa | E coli | S marcescens | P putida | S aureus | C albicans | Pen expansum |
| 0.05% DBHB | 3 hours | 3 hours | 3 hours | 3 hours | 1 hour | 3 hours | 4 days |
| 0.10% DBHB | 3 hours | 3 hours | 3 hours | <1 hour | <1 hour | <1 hour | 2 days |
| 0.15% DBHB | <1 hour | <1 hour | 1 hour | <1 hour | <1 hour | <1 hour | 1 day |
| Control (no preservative) | >28 days | >28 days | >28 days | >28 days | >28 days | >28 days | >28 days |

EXAMPLE 3

Many antimicrobial preservatives are inactivated by polyalkoxylated nonionic emulsifiers which are widely used in emulsification of cosmetic, pharmaceutical and industrial emulsions. An illustrative study shows the effectiveness of a compound conforming to the general formula in killing normally resistant Gram negative bacteria in the presence of a polyalkoxylated nonionic emulsifier, namely Polysorbate 80, at 5% and 10% concentrations in water. Normal use levels of Polysorbate 80 are 1-5%.

3,5-Dibromo-4-hydroxybenzaldehyde was evaluated at 0.05%, 0.10% and 0.15% in 5% and 10% Polysorbate 80 solutions by a challenge test of the type described in Example 2.

| Test organisms | Initial inoculum level colony forming units per g |
|---|---|
| Pseudomonas aeruginosa | $5.7 \times 10^7$ |
| Pseudomonas putida | $4.3 \times 10^7$ |
| Serratia marcescens | $8.1 \times 10^7$ |
| Klebsiella oxytoca | $7.3 \times 10^7$ |
| Escherichia coli | $6.6 \times 10^7$ |

| Test system | Polysorbate 80 level % | Time taken to achieve total lethal effect against: P aeruginosa | P putida | S marcescens | K oxytoca | E coli |
|---|---|---|---|---|---|---|
| 0.05% DBHB | 5 | 3 hours | 3 hours | 1 day | 6 hours | 6 hours |
| 0.10% DBHB | 5 | <1 hour | <1 hour | 1 hour | 1 hour | <1 hour |
| 0.15% DBHB | 5 | <1 hour | <1 hour | 1 hour | <1 hour | <1 hour |
| Control | 5 | >28 days | >28 days | >28 days | >28 days | >28 days |
| 0.05% DBHB | 10 | 1 day | 1 hour | 1 day | 3 hours | 3 hours |
| 0.10% DBHB | 10 | 3 hours | 1 hour | 3 hours | 1 hour | 1 hour |
| 0.15% DBHB | 10 | <1 hour | <1 hour | 1 hour | <1 hour | <1 hour |
| Control | 10 | >28 days | >28 days | >28 days | >28 days | >28 days |

## EXAMPLE 4

A sample of a commercial shampoo preparation (without preservative) was divided into three portions and, to a first portion of this shampoo, the following preservative system was added:

0.3% (w/w) of a 30% w/w solution of DCHB in propylene glycol (resulting in a concentration of 0.09% of active DCHB).

To a further portion of the unpreserved shampoo, 0.3% (w/w) propylene glycol alone was added to ensure any observed bactericidal effect was due to DCHB.

20g portions of the unpreserved shampoo and the shampoo with the above additions were inoculated with 0.4 ml of a mixed culture of 6 species of Gram negative bacteria (including an isolate from a contaminated shampoo) to give an initial inoculum level of 2.14 x 107 colony forming units per g.

The inoculated samples were mixed by Vortex mixer and stored at room temperature. At regular timed intervals after challenge the total number of survivors in each sample portion were determined by serial dilution and plate counting (Nutrient Agar, 32°C) with the following results:

| Sample | Gram negative bacteria - Mixed Culture Colony forming units/g after: 3 hours | 1 day | 7 days | 14 days | 28 days |
|---|---|---|---|---|---|
| 1. Commercial shampoo, unpreserved | $2.1x10^7$ | $5.7x10^7$ | $2.5x10^6$ | $1.4x10^6$ | $4.1x10^6$ |
| 2. Commercial shampoo, + 0.3% of 30% DCHB in propylene glycol | $9.0x10^6$ | <10 | <10 | <10 | <10 |
| 3. Commercial shampoo + 0.3% propylene glycol | 2.1x107 | $3.8x10^7$ | $5.1x10^6$ | $2.0x10^6$ | $4.8x10^6$ |

The shampoo sample containing 0.3 % of 30 % DCHB in propylene glycol demonstrated a rapid and satisfactory bactericidal effect against these normally resistant gram negative bacteria, whereas the product unpreserved and product + propylene glycol alone showed a high level of bacterial survival.

EXAMPLE 5

A shampoo formulation was prepared to the following formula:

| | |
|---|---|
| Coconut diethanolamide | 2% |
| Sodium lauryl ethyl sulphate | 8% |
| Sterile water | 90% by weight |

Various additions were made to portions of this shampoo in sterile bottles under aseptic conditions, as follows:

1. 0.20% of 30% DCHB in propylene glycol (0.06% active DCHB)
2. 0.20% of 30% DCHB in propylene glycol
   + 0.05% citric acid
3. 0.05% citric acid (control 1)
4. No addition (control 2)

Each of these systems was inoculated with a mixed culture of 7 strains of Gram negative bacteria, at an initial inocullum level of 9.0x107 colony forming units per g. At 30 mins, 1 hour, 2 hours, 6 hours, 24 hours and 7 days after inoculation the total number of survivors in each sample portion were determined by serial dilution and plate counting; with the following results:

| Shampoo Containing | Mixed culture - colony forming units/g after: 30 mins | 1 hour | 2 hours | 6 hours | 24 hours | 7 days |
|---|---|---|---|---|---|---|
| 1. 0.20% of 30% DCHB in propylene glycol | 3.9x106 | $3.0\times10^{6}$ | $6.6\times10^{5}$ | $7.0\times10^{5}$ | $8.4\times10^{4}$ | 10 |
| 2. As 1 + 0.05% citric acid | 2.3x103 | 120 | <10 | <10 | <10 | <10 |
| 3. 0.05% citric acid (Control 1) | 9.0x107 | $3.5\times10^{7}$ | $6.5\times10^{7}$ | $>1\times10^{8}$ | $>1\times10^{8}$ | $>1\times10^{8}$ |
| 4. No addition (Control 2) | 9.0x107 | $9.5\times10^{7}$ | $>1\times10^{8}$ | $>1\times10^{8}$ | $>1\times10^{8}$ | $>1\times10^{8}$ |

CLAIMS

1. A method of preventing the growth of fungi and bacteria including Gram-negative bacteria in water-containing compositions characterised by the addition to the composition, as an agent for the control of Gram-negative bacteria, of one or more compounds of the general formula:

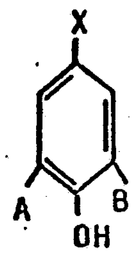

where X represents CHO or a mannic base, represented by

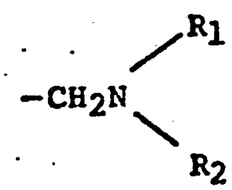

where $R_1$ and $R_2$ each represent an alkyl group (preferably containing 1 to 4 carbon atoms), or, together with an oxygen atom form the residue of an N-morpholinyl ring,

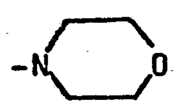

A represents fluorine, bromine, chlorine, iodine, an alkyl group containing from 1 to 4 carbon atoms, an alkoxy group containing from 1 to 4 carbon atoms, a hydroxyl group, a nitro group, a primary amino group or hydrogen;

B represents fluorine, bromine, chlorine, iodine, an alkyl group containing 1 to 4 carbon atoms, an alkoxy group containing 1 to 4 carbon atoms, a hydroxy group, a nitro group, a primary amino group or hydrogen provided that where X is a mannic base only one of A and B may represent hydrogen.

2. A method as claimed in claim 1 characterised in that X is -CHO and only one of A and B is hydrogen.

3. A method as claimed in claim 1 or 2 characterised in that the control agent of the general formula set forth in claim 1 is added to the water-containing compositions in an amount to provide from about 0.01% to about 0.6% by weight of the control agent based on the total weight of the water-containing composition.

4. A method as claimed in claim 3 characterised in that the control agent is provided in amount of from 0.02 to 0.2% by weight based on the total weight of the water-containing composition.

5. A method as claimed in any of claims 1 to 4 characterised in that the control agent comprises one or more of:-

(i) 3,5-Dibromo-4-hydroxybenzaldehyde

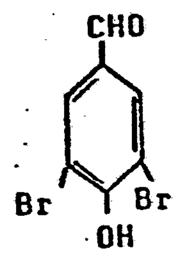

(ii) 3,5,-Dimethyl-4-hydroxybenzaldehyde

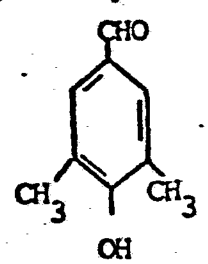

(iii) 3-Chloro-4-hydroxybenzaldehyde

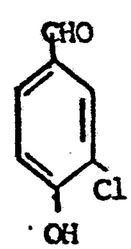

(iv) 3,5-Dimethyoxy-4-hydrozybenzaladehyde

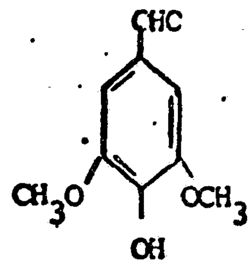

(v) 2,6-Dimethoxy-4-(N-morpholinyl) methyl phenol

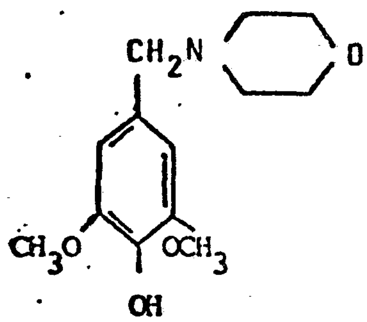

(vi) 3,5-Dichloro-4-hydroxybenzaldehyde

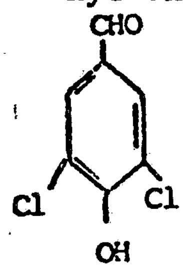

6. The use of one or more compounds of the general formula:

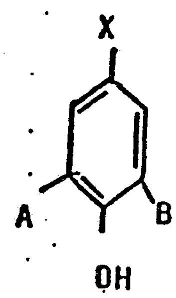

wherein X, A and B have the meanings defined in claim 1 in the preparation of a medicament for use in the control of bacteria (especially gram-negative bacteria) upon skin (especially human skin) by the application of said medicament to the skin.

7. A method as claimed in any of claims 1 to 5 characterised in that in addition to a compound of the

general formula set forth in claim 1 a potentiator for the antimicrobial activity of said compound is also added to the water-containing composition.

8. A method as claimed in claim 6 characterised in that the potentiator is a chelating agent for the compound of the general formula set forth in claim 1.

9. A method as claimed in claim 7 characterised in that the potentiator is citric acid or ethylene diamine tetraacetic acid.

10. A method as claimed in claims 1 to 8 in which the control agent is 3,5-dichloro-4-hydroxybenzaldehyde.

..........................................................

European Patent Office

# EUROPEAN SEARCH REPORT

0193257

Application number

EP 86 30 0284

## DOCUMENTS CONSIDERED TO BE RELEVANT

| Category | Citation of document with indication, where appropriate, of relevant passages | Relevant to claim |
|---|---|---|
| X | US-A-4 201 765 (S. SICHAK)<br>* Column 2, lines 25-34 *<br>--- | 1-9 |
| X | CHEMICAL ABSTRACTS, vol. 96, no. 21, 24th May 1982, page 405, no. 177786u, Columbus, Ohio, US; D. REHN et al.: "Antimicrobial activity of substituted aromatic aldehydes", & ZENTRALBL. BAKTERIOL., MIKROBIOL. HYG., ABT. 1, ORIG. B 1981, 172(6), 508-19<br>* Whole abstract *<br>--- | 1-9 |
| X | DIE PHARMAZIE, vol. 25, no. 8, August 1970, pages 480-484, Berlin, DD; W. WEUFFEN et al.: "Zusammenhänge zwischen chemischer Konstitution und keimwidriger Wirkung"<br>* Page 482, numbers 18,35,36 *<br>--- | 1-9 |
| X | JOURNAL OF THE CHEMICAL SOCIETY, Part I, 1963, pages 168-173, The Chemical Society, London, GB; K. CLARKE et al.: "The mechanism of the antibacterial action of phenols and salicylaldehydes. Part I"<br>* Page 169, table I: compounds 3,4 *<br>--- -/- | 1-9 |

CLASSIFICATION OF THE APPLICATION (Int. Cl.4)

A 01 N 33/10
A 01 N 35/04
A 61 K 7/00
A 61 K 7/06
A 61 K 7/48
A 61 K 31/11
A 61 K 31/135

TECHNICAL FIELDS SEARCHED (Int. Cl.4)

A 01 N
A 61 K

The present search report has been drawn up for all claims

| Place of search | Date of completion of the search | Examiner |
|---|---|---|
| THE HAGUE | 16-05-1986 | DECORTE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0193257
Application number

EP 86 30 0284

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | US-A-3 824 190 (M.W. WINICOW et al.)<br>* Column 6, lines 33-36£ *<br>--- | 7-9 | |
| A | CH-A- 338 847 (TH. GOLDSCHMIDT)<br>--- | | |
| A | GB-A-2 075 560 (L'OREAL)<br>----- | | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 16-05-1986 | DECORTE D. |

CATEGORY OF CITED DOCUMENTS

X particularly relevant if taken alone
Y particularly relevant if combined with another document of the same category
A technological background
O non-written disclosure
P intermediate document

T : theory or principle underlying the invention
E : earlier patent document, but published on, or after the filing date
D : document cited in the application
L : document cited for other reasons

& : member of the same patent family, corresponding document